# EUROPEAN PATENT SPECIFICATION

(11) **EP 4 082 622 B1**
(45) Date of publication and mention of the grant of the patent: **18.12.2024**
(21) Application number: 20905346.1
(22) Date of filing: 28.12.2020
(51) Int. Cl.: A61Q 13/00, A61K 8/11, A61K 8/58, B01J 13/14, C11B 9/00, B01J 13/18, B01J 13/22, C09B 67/02

(54) **METHOD FOR PRODUCING MICROCAPSULES**
VERFAHREN ZUM PRODUZIEREN VON MIKROKAPSELN
PROCÉDÉ DE PRODUCTION DE MICROCAPSULES

(30) Priority: 27.12.2019 JP 2019239908
(43) Date of publication of application: 02.11.2022
(73) Proprietor: Kao Corporation, Chuo-ku Tokyo 103-8210 (JP)
(72) Inventor: MOROFUJI, Tatsuya, Wakayama-shi, Wakayama 640-8580 (JP); YAMAZAKI, Daisuke, Wakayama-shi, Wakayama 640-8580 (JP)
(74) Representative: Hoffmann Eitle
(86) International application number: PCT/JP2020/049257
(87) International publication number: WO 2021/132728

(56) References cited:
- EP-A1- 3 078 415
- JP-A- 2009 221 059
- JP-A- 2013 237 051
- JP-A- 2015 128 762
- JP-A- 2017 114 802
- US-A1- 2010 247 660

## Description

### FIELD OF THE INVENTION

The present invention relates to a method for producing microcapsules.

### BACKGROUND OF THE INVENTION

Hitherto, it has been attempted that fragrance materials or medical ingredients or the like are encapsulated in microcapsules, and prolonged their effects by being incorporated in products. In particular, one of important properties of fabric treatment products, cosmetics and detergents, etc. is to impart fragrance to clothes or human body, and therefore there is an increasing demand for products having a high persistence of fragrance. For such a demand, synthesis of microcapsules by a sol-gel method is being studied.

For example, US2010/0247660A (PTL 1) discloses a method for preparing microcapsule particles containing an active substance in the core, which includes a step of adding a mixture of a sol-gel precursor and an aromatic oil to a surfactant solution, a step of homogenizing the resultant mixture of the sol-gel precursor, the aromatic oil and the surfactant solution, a step of curing the mixture to form microcapsule particles, and a step of modifying the microcapsule particles by second spherical complex formation to form coated microcapsule particles.

JP2013-237051A (PTL 2) discloses a method for preparing nanocapsules each having a core-shell structure, which includes (a) a step of emulsifying an oily phase containing a core material in an aqueous phase under high shearing force to prepare an oil-in-water emulsion, in which one or both of the oily phase and the aqueous phase contain a sol-gel precursor, (b) a step of high-pressure homogenization of the emulsion obtained in (a) to give a nanoemulsion, and (c) a step of hydrolyzing the sol-gel precursor and applying a condition for polycondensation thereto to give nanocapsules each having a metal oxide shell that encapsulates the core material therein, and the method further includes a step of chemically modifying the surface of the metal oxide shell.

JP2015-128762 (PTL 3) discloses a method for producing microcapsules aims at holding an organic compound of an active ingredient such as a fragrance material for long period of time, which includes a step (1) of emulsifying an organic phase containing at least one organic compound and a tetraalkoxysilane where the amount of the tetraalkoxysilane is 10% by mass or more and 60% by mass or less relative to the organic compound, in an aqueous phase containing a surfactant, followed by sol-gel reaction under an acidic condition to form capsules each having a core and a first shell, and a step (2) of further adding a tetraalkoxysilane to the aqueous dispersion containing the capsules formed in the step (1) followed by sol-gel reaction in such a manner that the initial pH of the sol-gel reaction of the step (2) is kept lower than the initial pH of the sol-gel reaction in the step (1), thereby forming capsules each having a second shell that encapsulates the first shell.

EP 3 078 415 A1, a family member of JP2015-128762, discloses a method for producing microcapsules comprising emulsifying an oil phase consisting of 40 g of model fragrance material A and 10 g of TEOS in an aqueous solution consisting of 3.48 g of Quartamin 60W and 196.52 g of ion-exchanged water. The pH of said emulsion is adjusted to 3.7 using a 1N sodium hydroxide aqueous solution after which 6.05 g of TEOS is added in a second step. The total amount of model fragrance A and TEOS must therefore be less than (40+10)/(40+10+3.48+196.52)*100%=20% of the aqueous dispersion before addition of the 6.05 of TEOS in the second step.

### SUMMARY OF THE INVENTION

The present invention relates to a method for producing microcapsules each having a shell that contains silica as a constituent ingredient, and a core that contains an organic compound inside the shell, which includes:
a step 1 of emulsifying an organic phase containing an organic compound and a tetraalkoxysilane in an aqueous phase containing a surfactant, followed by sol-gel reaction under an acidic condition to form capsules (1) each having a core and a shell, thereby giving an aqueous dispersion that contains the capsules (1), and
a step 2 of diluting the aqueous dispersion prepared in the step 1 with water added thereto, and further adding a tetraalkoxysilane and subjecting the obtained mixture to sol-gel reaction to form a shell that encapsulates the capsule (1), thereby giving microcapsules, and wherein:
   the dilution in the step 2 is carried out in such a manner that the total amount of the organic compound and the tetraalkoxysilane in the step 1 is 18% by mass or less relative to 100% by mass of the total amount of the aqueous dispersion before addition of tetraalkoxysilane in the step 2.

### BRIEF DESCRIPTION OF THE DRAWINGS

Fig. 1 is a scanning electron micrograph (SEM) of microcapsules obtained in Example 2.
Fig. 2 is a scanning electron micrograph (SEM) of microcapsules obtained in Comparative Example 1.

### DETAILED DESCRIPTION OF THE INVENTION

In the techniques of PTLs 1 to 3, the long-term retentiveness of organic compounds, which are active ingredients such as a fragrance material of microcapsules, is not sufficient, and further improvement is required.

The present invention relates to a method for producing microcapsules capable of holding an organic compound as an active ingredient such as a fragrance material encapsulated therein for a long period of time.

The present inventor has found that, by forming microcapsules via a specific two-stage process, an organic compound as an active ingredient such as a fragrance material encapsulated therein can be held for a long period of time. Namely, the present invention relates to a method for producing microcapsules each having a shell that contains silica as a constituent ingredient, and a core that contains an organic compound inside the shell, which includes:
a step 1 of emulsifying an organic phase containing an organic compound and a tetraalkoxysilane in an aqueous phase containing a surfactant, followed by sol-gel reaction under an acidic condition to form capsules (1) each having a core and a shell, thereby giving an aqueous dispersion that contains the capsules (1), and
a step 2 of diluting the aqueous dispersion prepared in the step 1 with water added thereto, and further adding a tetraalkoxysilane and subjecting the obtained mixture to sol-gel reaction to form a shell that encapsulates the capsule (1), thereby giving microcapsules, and wherein:
   the dilution in the step 2 is carried out in such a manner that the total amount of the organic compound and the tetraalkoxysilane in the step 1 is 18% by mass or less relative to 100% by mass of the total amount of the aqueous dispersion before addition of tetraalkoxysilane in the step 2.

According to the present invention, there can be provided a method for producing microcapsules capable of holding an organic compound as an active ingredient such as a fragrance material encapsulated therein for a long period of time.

### [Production Method for Microcapsules]

The production method for microcapsules of the present invention is a method for producing microcapsules each having a shell that contains silica as a constituent ingredient, and a core that contains an organic compound inside the shell, which includes a step 1 of emulsifying an organic phase containing an organic compound and a tetraalkoxysilane in an aqueous phase containing a surfactant, followed by sol-gel reaction under an acidic condition to form capsules (1) each having a core and a shell, thereby giving an aqueous dispersion that contains the capsules (1) (hereinafter this may be simply referred to as "step 1"), and a step 2 of diluting the aqueous dispersion prepared in the step 1 with water added thereto, and further adding a tetraalkoxysilane and subjecting the obtained mixture to sol-gel reaction to form a shell that encapsulates the capsule (1), thereby giving microcapsules (hereinafter this may be simply referred to as "step 2"), and wherein the dilution in the step 2 is carried out in such a manner that the total amount of the organic compound and the tetraalkoxysilane in the step 1 is 18% by mass or less relative to 100% by mass of the total amount of the aqueous dispersion before addition of tetraalkoxysilane in the step 2.

In the present invention, the "sol-gel reaction" means a reaction of forming silica that is a constituent ingredient of a shell by hydrolysis and polycondensation reaction of a tetraalkoxysilane (namely, silica precursor) via a sol and gel state. Specifically, a tetraalkoxysilane is hydrolyzed and the silanol compound forms a siloxane oligomer by dehydrating condensation and dealcoholating condensation, and via further dehydrating condensation, silica is formed.

In the present invention, in the case where all the amount of the aqueous dispersion obtained in the step 1 is subjected to the step 2, the amount of the organic compound and the tetraalkoxysilane in the step 1 is the amount of the organic compound and the tetraalkoxysilane used in the step 1. In the case where a part of the aqueous dispersion obtained in the step 1 is subjected to the step 2, the amount of the organic compound and the tetraalkoxysilane in the step 1 is an amount obtained by proportional conversion from the amount of the organic compound and the tetraalkoxysilane used in the step 1, using the amount of the aqueous dispersion obtained in the step 1 that is subjected to the step 2.

In the present specification, the long-term retentiveness of the encapsulated organic compound may be referred to as "long-term retentiveness". Also, the shell formed in the step 1 may be referred to as "first shell" and the shell formed in the step 2 as "second shell".

The microcapsules obtained according to the production method of the present invention can hold an organic compound encapsulated therein, especially an active ingredient such as a fragrance material, for a long period of time. Though not clear, the reason can be considered as follows.

In the step 1, the solubility of tetraalkoxysilane in the aqueous phase is low. Tetraalkoxysilane is gradually partitioned in the aqueous phase, and forms an alcohol such as ethanol by hydrolysis, and forms a silica shell by condensation. At that time, when the concentration of the oily phase containing an organic compound is too low, tetraalkoxysilane is excessively partitioned in the aqueous phase so that a useless silica sol not adsorbing to the oily phase component increases inefficiently.

On the other hand, after the sol-gel reaction in the step 1, the solubility of tetraalkoxysilane in the aqueous phase becomes high by the alcohol formed by hydrolysis, and therefore becomes an unsuitable state for efficient silica shell formation. Therefore, water is added to the aqueous dispersion obtained in the step 1 for dilution so that the solubility of tetraalkoxysilane in the aqueous phase in the step 2 can be controlled in an appropriate range. Further, it is considered that, by reducing the ion intensity in the aqueous phase, silica shell formation by the sol-gel reaction in the step 2 can be carried out efficiently and therefore microcapsules each having a dense and strong shell can be obtained. As a result, it is considered that the long-term retentiveness of an organic compound as an active ingredient such as a fragrance material can be thereby improved.

### <Step 1>

The step 1 is a step of emulsifying an organic phase containing an organic compound and a tetraalkoxysilane in an aqueous phase containing a surfactant, followed by sol-gel reaction under an acidic condition to form capsules (1) each having a core and a shell, thereby giving an aqueous dispersion that contains the capsules (1).

Tetraalkoxysilane for use in the step 1 is preferably one having an alkoxy group having 1 or more and 4 or less carbon atoms, more preferably at least one selected from the group consisting of tetramethoxysilane, tetraethoxysilane and tetraisopropoxysilane, even more preferably at least one selected from the group consisting of tetramethoxysilane and tetraethoxysilane, further more preferably tetraethoxysilane, from the viewpoint of promoting sol-gel reaction.

The amount of the tetraalkoxysilane in the step 1 is preferably 10% by mass or more, more preferably 12% by mass or more, even more preferably 14% by mass or more, further more preferably 18% by mass or more, further more preferably 20% by mass or more, relative to 100% by mass of the total amount of the organic compound, from the viewpoint of promoting sol-gel reaction to form a sufficiently dense shell, and is preferably 60% by mass or less, more preferably 50% by mass or less, even more preferably 40% by mass or less, further more preferably 35% by mass or less, further more preferably 30% by mass or less, relative to 100% by mass of the total amount of the organic compound, from the viewpoint of preventing excessive tetraalkoxysilane from remaining in the organic compound.

The core of the microcapsules in the present invention contains an organic compound. The organic compound may be kind alone or two or more kinds either singly or as combined.

The organic compound is preferably one or more selected from the group consisting of a fragrance material, a fragrance precursor, an oil, an antioxidant, a cooling sensation agent, a dye, a colorant, a silicone, a solvent and an oil-soluble polymer, more preferably one or more selected from the group consisting of a fragrance material, a fragrance precursor, an oil, an antioxidant and a solvent, even more preferably one or more selected from the group consisting of a fragrance material and a fragrance precursor.

The fragrance precursor includes a compound that reacts with water to release a fragrance component, and a compound that reacts with light to release a fragrance component.

The compound that reacts with water to release a fragrance component includes a silicate compound having an alkoxy component derived from a fragrance alcohol, a fatty acid ester compound having an alkoxy component derived from a fragrance alcohol, an acetal compound or a hemiacetal compound obtained by reaction of a carbonyl component derived from a fragrance aldehyde or a fragrance ketone and an alcohol compound, a Schiff base compound obtained by reaction of a carbonyl component derived from a fragrance aldehyde or a fragrance ketone and a primary amine compound, and a hemiaminal compound or hydrazone compound obtained by reaction of a carbonyl component derived from a fragrance aldehyde or a fragrance ketone and a hydrazine compound.

The compound that reacts with light to release a fragrance component includes a 2-nitrobenzyl ether compound having an alkoxy component derived from a fragrance alcohol, an α-keto-ester compound having a carbonyl component derived from a fragrance aldehyde or fragrance ketone, and a coumarate compound having an alkoxy compound derived from a fragrance alcohol. These fragrance precursors can be used as a polymer of, for example, a reaction product of some carboxy groups of a polyacrylic acid and a fragrance alcohol.

Among these, a compound that reacts with water to release a fragrance component is preferred, and a silicate compound having an alkoxy component derived from a fragrance alcohol is more preferred.

A calculated value of a common logarithm "LogP" of a partition coefficient P between n-octanol and water (n-octanol/water) of the organic compound (hereinafter also referred to as "CLogP value") is preferably 1 or more, more preferably 2 or more, even more preferably 3 or more, and is preferably 30 or less, more preferably 20 or less, even more preferably 10 or less. When the CLogP value of the organic compound is 1 or more, the ratio of encapsulating an organic compound within the microcapsules (hereinafter also referred to as "encapsulation ratio") increases. Here, the same may apply to the case of a fragrance material composition containing plural fragrance material components and oils, that is, when the CLogP value of the fragrance material composition is 1 or more, the encapsulation ratio of the fragrance material composition in the resultant microcapsules can increase.

Here, a LogP value is an index to indicate the hydrophilicity or hydrophobicity of a compound, and the CLogP value is a "calculated LogP (ClogP)" that is calculated according to the method described in A. Leo Comprehensive Medicinal Chemistry, Vol. 4 C. Hansch, P. G. Sammens, J. B Taylor and C. A. Ramsden, Eds., p. 295, Pergamon Press, 1990, and is a CLogP value calculated by Program CLOGP v. 4.01. For example, in the case where the organic compound is a fragrance material composition containing plural fragrance material components and oils, the CLogP value of the fragrance material composition can be determined by multiplying the CLogP value of each fragrance material component and oil by the volume ratio of thereof in the fragrance material composition, and summing up the resultant data.

The surfactant used in the step 1 is preferably a cationic surfactant.

The cationic surfactant includes compounds containing a nitrogen-based cationic group, and surfactants that tend to be cationic by controlling the pH thereof. Specific examples include alkylamine salts and quaternary ammonium salts. The quaternary ammonium salts include alkyl quaternary ammonium salts such as alkyltrimethylammonium salts, dialkyldialkylammonium salts, and alkylbenzyldimethylammonium salts.

The carbon number of the alkyl group of the alkylamine salts and alkyl quaternary ammonium salts is preferably 10 or more, more preferably 12 or more, even more preferably 14 or more, and is preferably 24 or less, more preferably 22 or less, even more preferably 20 or less, further more preferably 18 or less.

The alkylamine salts include laurylamine acetate, and stearylamine acetate. The quaternay ammonium salts include alkyl quaternay ammonium salts, such as alkyltrimethylammonium chlorides such as lauryltrimethylammonium chloride, stearyltrimethylammonium chloride, and cetyltrimethylammonium chloride; dialkyldimethylammonium chlorides such as distearyldimethylammonium chloride; and alkylbenzyldimethylammonium chlorides. Above all, quaternay ammonium salts are preferred, alkyl quaternary ammonium salts are more preferred, alkyltrimethylammonium chlorides are even more preferred, and cetyltrimethylammonium chloride is further more preferred.

The concentration of the surfactant in the aqueous phase in the step 1 is preferably 0.2% by mass or more, more preferably 0.3% by mass or more, even more preferably 0.4% by mass or more, and is preferably 10% by mass or less, more preferably 5% by mass or less, even more preferably 2% by mass or less, further more preferably 1% by mass or less.

In the step 1, an organic phase is emulsified in the aqueous phase to give an emulsion. Here, the organic phase may be any phase that contains the above-mentioned organic compound and tetraalkoxysilane. The mixing ratio of the organic phase to the aqueous phase is preferably 3% by mass or more, more preferably 5% by mass or more, even more preferably 7% by mass or more, further more preferably 10% by mass or more, further more preferably 20% by mass or more, and is preferably 70% by mass or less, more preferably 50% by mass or less, even more preferably 45% by mass or less, further more preferably 40% by mass or less, as the concentration of an organic phase in the emulsion, from the viewpoint of productivity and the viewpoint of producing a stable emulsion.

Not specifically limited, the stirring means for use in preparing the emulsion may be a homogenizer having a strong shear force, a high-pressure disperser, and an ultrasonic disperser. In addition, a homomixer, "DISPER" (tradename, by PRIMIX Corporation), "CLEAMIX" (tradename, by M Technique Co., Ltd.), and "CAVITRON" (tradename, by Pacific Machinery & Engineering Co., Ltd.) can also be used.

The volume-average particle diameter of the emulsified drops in the emulsion in the step 1 is preferably 0.1 pm or more, more preferably 0.5 pm or more, even more preferably 1 pm or more, and is preferably 50 pm or less, more preferably 10 pm or less, even more preferably 5 pm or less, further more preferably 3 pm or less, from the viewpoint of obtaining microcapsules having a desired particle size. In the present invention, the volume-average particle diameter of the emulsified drops of the emulsion can be measured according to the method described in the section of Examples.

The sol-gel reaction in the step 1 is carried out under an acidic condition. The initial pH in the sol-gel reaction is preferably 3.5 or more, more preferably 3.7 or more, and is preferably 4.3 or less, more preferably 4.0 or less.

pH control can be attained using an acidic solution or an alkaline solution.

The acidic solution includes an inorganic acid such as hydrochloric acid, nitric acid, sulfuric acid and phosphoric acid; an organic acid such as acetic acid and citric acid; and a liquid prepared by adding a cation exchange resin to water or methanol. Preferred is hydrochloric acid or citric acid. The alkaline solution includes a sodium hydroxide solution, a sodium hydrogencarbonate solution, a potassium hydroxide solution, and an ammonium hydroxide solution. Preferred is a sodium hydroxide solution or a sodium hydrogencarbonate solution.

The pH of the emulsion may be lower than a desired numerical value. In such a case, preferably, the emulsion is controlled using the above-mentioned alkaline solution.

The reaction temperature of the sol-gel reaction in the step 1 is preferably 5°C or higher, more preferably 10°C or higher, even more preferably 15°C or higher, and is preferably 70°C or lower, more preferably 50°C or lower, even more preferably 40°C or lower.

The reaction time of the sol-gel reaction in the step 1 can be appropriately controlled by the production scale. In the case where the time at which the inside of the reaction system can reach a predetermined temperature is defined as a reaction start, the reaction time is preferably 0.5 hours or more, more preferably 1 hour or more, even more preferably 5 hours or more, and is preferably 50 hours or less, more preferably 40 hours or less, even more preferably 30 hours or less.

### <Step 2>

The step 2 is a step of diluting the aqueous dispersion prepared in the step 1 with water added thereto, and further adding a tetraalkoxysilane and subjecting the obtained mixture to sol-gel reaction to form a shell that encapsulates the capsule (1), thereby giving microcapsules.

The tetraalkoxysilane used in the step 2 is preferably one having an alkoxy group having 1 or more and 4 or less carbon atoms, more preferably at least one selected from the group consisting of tetramethoxysilane, tetraethoxysilane and tetraisopropoxysilane, even more preferably at least one selected from the group consisting of tetramethoxysilane and tetraethoxysilane, further more preferably tetraethoxysilane, from the viewpoint of promoting sol-gel reaction.

The amount of the tetraalkoxysilane in the step 2 is preferably 10% by mass or more, more preferably 30% by mass or more, even more preferably 50% by mass or more, further more preferably 70% by mass or more, and is preferably 200% by mass or less, more preferably 150% by mass or less, even more preferably 110% by mass or less, further more preferably 95% by mass or less, relative to 100% by mass of the amount of the organic compound in the step 1.

In the present invention, the total addition amount of tetraalkoxysilane, that is, the total amount of tetraalkoxysilane used in the step 1 and the step 2 is preferably 30% by mass or more, more preferably 50% by mass or more, even more preferably 70% by mass or more, further more preferably 90% by mass or more, and is preferably 250% by mass or less, more preferably 200% by mass or less, even more preferably 150% by mass or less, further more preferably 130% by mass or less, relative to 100% by mass of the amount of the organic compound in the step 1. When the total addition amount of tetraalkoxysilane is controlled within the above range, the encapsulated organic compound can be held for a long period of time.

In the present invention, the dilution in the step 2 is such that the total amount of the organic compound and tetraalkoxysilane in the step 1 is 18% by mass or less, preferably 15% by mass or less, more preferably 10% by mass or less, relative to 100% by mass of the total amount of the aqueous dispersion before addition of tetraalkoxysilane in the step 2, from the viewpoint of improving the long-term retentiveness of organic compound, and is preferably 2% by mass or more, more preferably 3% by mass or more, even more preferably 4% by mass or more, further more preferably 5% by mass or more, relative to 100% by mass of the total amount of the aqueous dispersion before addition of tetraalkoxysilane in the step 2, from the viewpoint of production efficiency.

The total amount of the organic compound and tetraalkoxysilane in the step 1 relative to the total amount of the aqueous dispersion before addition of tetraalkoxysilane in the step 2 can be controlled by further diluting the aqueous dispersion obtained in the step 1 with water added thereto.

Namely, in the present invention, from the viewpoint of production efficiency, the aqueous dispersion obtained in the step 1 is diluted with water added thereto before addition of tetraalkoxysilane in the step 2.

The total amount of the organic compound and tetraalkoxysilane in the step 1 relative to 100% by mass of the total amount of the aqueous dispersion obtained in the step 1 before dilution is preferably 5% by mass or more, more preferably 10% by mass or more, even more preferably 15% by mass or more, and is preferably 50% by mass or less, more preferably 40% by mass or less.

The dilution ratio is preferably 2 times, more preferably 2.5 times, and is preferably 20 times or less, more preferably 10 times or less, even more preferably 7 times or less. The "dilution ratio" in the present specification is a ratio by mass of the total amount of the aqueous dispersion after dilution with water

(hereinafter also referred to as "diluted aqueous dispersion") to the total amount before dilution of the aqueous dispersion obtained in the step 1 to subjected to the step 2 (total amount of diluted aqueous dispersion/total amount before dilution of aqueous dispersion obtained in step 1 to be subjected to step 2).

In the present invention, in the step 2, an organic polymer compound may be further added to the aqueous dispersion obtained in the step 1.

Here, the organic polymer compound means a compound having a weight-average molecular weight of 5,000 or more.

The organic polymer compound is preferably at least one selected from the group consisting of a cationic polymer and a nonionic polymer.

The nonionic polymer means a water-soluble polymer having no charge in water. Using a nonionic polymer, the microcapsules in the present invention can be given functions in accordance with the use of the microcapsules. For example, in the case where the microcapsules in the present invention are used for a fiber treatment agent composition such as a softener composition, adsorbability to fibers can be improved.

In the present specification, "water-soluble polymer" means a polymer such that when the polymer is dried at 105°C for 2 hours and when the polymer having reached a constant mass is dissolved in 100 g of water at 25°C, the dissolution amount thereof is 1 mg or more.

The nonionic polymer includes a polymer having a structural unit derived from a nonionic monomer, water-soluble polysaccharides (e.g., cellulose-based, gum-based, starch-based, etc.), and derivatives thereof.

The nonionic monomer includes a (meth)acrylate having a hydrocarbon group derived from an aliphatic alcohol having 1 or more and 22 or less carbon atoms; a styrene-based monomer such as styrene; an aromatic group-containing (meth)acrylate such as benzyl (meth)acrylate; vinyl acetate; vinyl pyrrolidone; vinyl alcohol; a polyalkylene glycol (meth)acrylate such as polyethylene glycol mono(meth)acrylate; an alkoxy-polyalkylene glycol mono(meth)acrylate such as methoxypolyethylene glycol mono(meth)acrylate, and octoxypolyethylene glycol mono(meth)acrylate; and a (meth)acrylamide.

The nonionic polymer is preferably at least one selected from the group consisting of polyvinyl pyrrolidone, a copolymer of vinyl pyrrolidone and any other nonionic monomer such as vinyl pyrrolidone/vinyl acetate copolymer, and a cellulose-based polymer such as hydroxyethyl cellulose, hydroxypropyl cellulose, hydroxypropylmethyl cellulose, and hydroxyethylmethyl cellulose, more preferably at least one selected from the group consisting of polyvinyl pyrrolidone and hydroxypropyl cellulose.

The cationic polymer includes a polymer containing a quaternary ammonium base and in addition thereto, a polymer having a nitrogen-based cationic group, and a polymer that can be cationic by pH control. Using a cationic polymer, the microcapsules (1) formed in the step 1 can be relaxed from an aggregable state in an aqueous medium, and can be suppressed from formation of coarse particles in the subsequent step 2.

The cationic polymer includes polydiallyldimethylammonium chlorides and copolymers thereof such as poly(diallyldimethylammonium chloride), poly(acrylic acid-co-diallyldimethylammonium chloride), poly(acrylamide-co-diallyldimethylammonium chloride) and poly(acrylamide-co-acrylic acid-co-diallyldimethylammonium chloride), and poly(2-(methacryloyloxy)ethyltrimethylammonium chloride), polyethyleneimine, polyallylamine, cationized cellulose, cationized guar gum, cationized tara gum, cationized fenugreek gum and cationized locust bean gum. Among these, preferred are polydiallyldimethylammonium chlorides and copolymers thereof, more preferred is at least one compound selected from the group consisting of poly(diallyldimethylammonium chloride), poly(acrylic acid-co-diallyldimethylammonium chloride) and poly(acrylamide-co-acrylic acid-co-diallyl dimethylammonium chloride), and even more preferred is poly(diallyldimethylammonium chloride).

The cationic group equivalent of the cationic polymer is preferably 1 meq/g or more, more preferably 3 meq/g or more, even more preferably 4.5 meq/g or more, and is preferably 10 meq/g or less, more preferably 8 meq/g or less, from the viewpoint of dispersibility of the capsules (1) and from the viewpoint of preventing formation of coarse particles, and also from the viewpoint of improving the long-term retentiveness of organic compounds. The cationic polymer may contain an anionic group, and in such a case, the anionic group equivalent contained in the cationic polymer is preferably 3.5 meq/g or less, more preferably 2 meq/g or less, even more preferably 1 meq/g or less. In the present invention, the cationic group equivalent of the cationic polymer is one calculated based on the monomer composition.

The amount to be added of the organic polymer compound is preferably 0.05% by mass or more, more preferably 0.1% by mass or more, even more preferably 0.2% by mass or more, and is preferably 5% by mass or less, more preferably 3% by mass or less, even more preferably 2% by mass or less, relative to 100% by mass of the amount of the organic compound in the step 1.

In the step 2 where the organic polymer compound is further added, the compound is added preferably as an aqueous solution at the time when water is added for dilution in the step 2.

The reaction temperature of the sol-gel reaction in the step 2 is preferably 5°C or higher, more preferably 10°C or higher, even more preferably 15°C or higher, and is preferably 70°C or lower, more preferably 50°C or lower, even more preferably 40°C or lower.

The method of adding tetraalkoxysilane in the step 2 is not specifically limited, and it may be added all at a time, or may be dropwise added taking a predetermined period of time, but from the viewpoint of preventing formation of coarse grains to improve the long-term retentiveness of organic compound, dropwise addition is preferred.

The time to dropwise adding tetraalkoxysilane in the step 2 can be appropriately controlled depending on the production scale and the like, and is preferably 10 minutes or more, more preferably 60 minutes or more, even more preferably 100 minutes or more, and is preferably 1,000 minutes or less, more preferably 700 minutes or less, even more preferably 500 minutes or less.

In the case where tetraalkoxysilane is dropwise added in the step 2, preferably, the reaction is further continued after the addition. The reaction time of the sol-gel reaction after the addition can be appropriately controlled depending on the production scale and the like, and is preferably 0.5 hours or more, more preferably 1 hour or more, even more preferably 5 hours or more, and is preferably 50 hours or less, more preferably 40 hours or less, even more preferably 30 hours or less.

The microcapsules formed in the step 2 are obtained in a dispersed state in water. Depending on use, these can be used directly as such, but the microcapsules may be separated and collected. For the separation method, employable is a filtration method or a centrifugation method.

### [Microcapsules]

The microcapsules produced by the method of the present invention are microcapsules each having a shell containing silica as a constituent ingredient, and a core containing the above-mentioned organic compound inside the shell.

The average thickness of the shell (first shell) formed in the step 1 is preferably 5 nm or more, and is preferably 20 nm or less, more preferably 15 nm or less. The first shell is preferably a dense layer with as few pores as possible, from the viewpoint of improving the long-term retentiveness of organic compound.

The average thickness of the shell (second shell) formed in the step 2 is preferably 10 nm or more, more preferably 20 nm or more, and is preferably 100 nm or less, more preferably 80 nm or less.

The average thickness of the first shell and the second shell of the microcapsules can be measured by transmission electron microscopy (TEM). Specifically, under transmission electron microscope observation, the thickness of the first shell and the second shell is measured on the photographic image. This operation is carried out in different five view fields. From the resultant data, the average thickness distribution of the first shell and the second shell is determined. As a rough indication, the transmission electron microscope has a magnification of 10,000 times or more and 100,000 times or less, but can be appropriately controlled depending on the size of the microcapsules. Here, as a transmission electron microscope, for example, "JEM-2100" (tradename by JEOL Corporation) can be used.

The volume-average particle diameter of the microcapsules produced by the method of the present invention is preferably 0.1 pm or more, more preferably 0.2 pm or more, even more preferably 0.5 pm or more, from the viewpoint of increasing the encapsulation ratio of core to improve the long-term retentiveness of organic compound, and is preferably 50 pm or more, more preferably 10 pm or more, even more preferably 3 pm or more, from the viewpoint of improving the physical strength of microcapsules to improve the long-term retentiveness of organic compound.

The volume-average particle diameter of the microcapsules produced by the method of the present invention can be measured, using a laser diffraction/scattering particle size distribution analyzer "LA-950" (trade name by Horiba, Ltd.). In that case, the measurement is performed using a flow cell, and water is used as a dispersion medium. A refractive index is set to 1.45-0-i for a dispersoid. A suspension containing microcapsules is added to the flow cell, and the measurement is carried out at a concentration at which a transmittance thereof is near 90%, to thereby determine the average particle diameter based on the volume.

The microcapsules produced by the method of the present invention can be used in various applications, for example, as cosmetic materials such as milk, cosmetic fluid, lotion, essence liquid, cream, gel formulation, hair treatment agent, and quasi-drugs, fiber treatment agents such as detergent, softener, and antiwrinkle spray, hygiene products such as paper diaper, and aromatic materials.

The microcapsules produced by the method of the present invention can be used as contained or blended in a composition such as a detergent composition, a fiber treatment composition, a cosmetic composition, an aromatic composition, and a deodorant composition. The composition is preferably a detergent composition such as a powdery detergent composition, and a liquid detergent composition; and a fiber treatment composition such as a softener composition, more preferably a fiber treatment composition, even more preferably a softener composition.

### EXAMPLES

Various measurements in Examples and Comparative Examples were according to the following methods.

### (1) Volume-Average Particle Diameter

The volume-average particle diameter of the emulsified drops in the emulsion in the step 1 and the microcapsules were measured using a laser diffraction/scattering particle size distribution analyzer "LA-950" (trade name by Horiba, Ltd.). The measurement was performed using a flow cell, and water was used as a dispersion medium. A refractive index was set to 1.45-0-i for a dispersoid. The emulsion prepared in the step 1 or the suspension containing microcapsules formed in the step 2 was added to the flow cell, and the measurement was carried out at a concentration at which a transmittance thereof was near 90%, to determine the average particle diameter based on the volume.

### (2) Encapsulation Ratio of Fragrance Material in Microcapsules

50 mg of the suspension containing microcapsules obtained in the step 2 were dispersed in 5 mL of acetonitrile containing tridecane in a concentration of 10 pg/mL, as an internal standard, then the resultant dispersion was irradiated with ultrasonic waves for 60 minutes using an ultrasonic irradiator (by Branson Corporation, Model "5510") under the condition of an output of 180 W and an oscillation frequency of 42 kHz, and made to pass through a membrane filter (by Toyo Roshi Kaisha, Ltd., tradename "DISMIC", Model Code "13JP020AN"), and thereafter the fragrance material contained in the dispersion was identified through gas chromatography to determine the amount of the fragrance material component included in the suspension.

On the other hand, 20 g of the suspension containing microcapsules obtained in the step 2 was made to pass through a membrane filter (by Millipore Corporation, tradename "Omnipore", Model Code "JAWP04700") to thereby collect microcapsules on the membrane filter. Further, on the membrane filter, the microcapsules were washed with 10 mL of ion-exchanged water and then 10 mL of hexane, and thereafter the microcapsules were immersed in 2 mL of acetonitrile containing tridecane in a concentration of 10 pg/ml, as an internal standard, and the resultant suspension was irradiated with ultrasonic waves for 60 minutes under the same condition as above, and again made to pass through a membrane filter (by Toyo Roshi Kaisha, Ltd., tradename "DISMIC", Model Code "13JP020AN"), and thereafter the fragrance material contained in the suspension was identified through gas chromatography to determine the amount of the fragrance material component encapsulated in the microcapsules.

From the amount of the fragrance material component contained in the suspension obtained in the above and the amount of the fragrance material component encapsulated in the microcapsules, the encapsulation ratio (%) of fragrance material was determined according to the following formula.

Encapsulation Ratio of Fragrance Material (%) = {(amount of fragrance material component encapsulated in microcapsules)/(amount of fragrance material component contained in suspension)] × 100

### (3) Observation with Scanning Electron Microscope (SEM)

0.05 g of the suspension containing microcapsules obtained in the step 2 was metered, diluted 100-fold with ion-exchanged water, and then filtered through a membrane filter (by Millipore Corporation, tradename "Omnipore", Model Code "JAWP04700"). The capsules on the membrane filter were transferred to a stage and dried on the stage. The microcapsules on the stage were processed for Au-Pd deposition, and then observed with a field emission-type scanning electron microscope "S-4000" (by Hitachi High Technologies Corporation).

### <Model Fragrance Material>

A model fragrance material A (volume-average CLogP: 4.2. specific gravity: 0.96) and a model fragrance material B (volume-average CLogP: 3.5, specific gravity: 0.87) having the composition shown in Table 1 and Table 2, respectively, were used as the organic compound to be encapsulated in microcapsules. The volume-average CLogP value of the model fragrance material was calculated by multiplying the CLogP values of all the fragrance components in the model fragrance material by the volume ratio thereof in the model fragrance material and summing up the resultant data.

**Table 1: Model Fragrance Material A**

| Compound Name | Content (mass%) | CLogP |
|---|---|---|
| Hexyl Acetate | 10 | 2.8 |
| Tetrahydrolinalol | 10 | 3.6 |
| Hexylcinnamaldehyde | 20 | 4.9 |
| Methyl Dihydrojasmonate | 10 | 3.0 |
| α-ionone | 10 | 3.9 |
| Lilial | 20 | 4.4 |
| Hexyl Salicylate | 20 | 5.1 |

**Table 2: Model Fragrance Material B**

| Compound Name | Content (mass%) | CLogP |
|---|---|---|
| Citronellol | 50 | 3.6 |
| Geraniol | 25 | 3.5 |
| Hexyl Butyrate | 5 | 3.8 |
| Linalyl Acetate | 20 | 3.3 |

### Example 1

### (Step 1)

1.67 g of ΓaUARTAMIN 60W (tradename by Kao Corporation, cetyltrimethylammonium chloride, active ingredient: 30% by mass) was diluted with 98.33 g of ion-exchanged water to give an aqueous phase. An organic phase prepared by mixing 40 g of the model fragrance material A and 10 g of tetraethoxysilane (hereinafter also referred to as "TEOS") was added to the aqueous phase, and the mixture liquid was emulsified using a homomixer set at a rotation number of 8,500 rpm. At that time, the volume-average particle diameter of the emulsified drops was 1.12 pm. This was controlled to have a pH of 3.9 using an aqueous 1N sodium hydroxide solution, then transferred to a separable flask equipped with a stirring blade and a cooling device, and while the liquid temperature was kept at 30°C, this was stirred at 160 rpm for 17 hours to give 150 g of an aqueous dispersion containing capsules (1-1) each having a core and a shell.

### (Step 2)

As a cationic polymer, Merquat 100 (tradename by Lubrizol Japan, poly(diallyldimethylammonium chloride), cation group equivalent 6.2 meq/g, active ingredient 42% by mass, weight-average molecular weight 150,000) was diluted with ion-exchanged water to prepare an aqueous solution of Merquat 100 containing 3% by mass of the active ingredient.

2 g of the aqueous 3 mass% Merquat 100 solution (active ingredient 0.06 g) was added to 25 g of the aqueous dispersion prepared in the step 1, and further diluted with 73 g of water added thereto (dilution ratio 4 times). Next, while the liquid temperature was kept at 30°C, 6.0 g of TEOS was dropwise added via a dropping pump "Atlas syringe pump" (tradename, by Syrris Corporation) taking 425 minutes. After the dropwise addition, this was kept stirred for further 18 hours, and then cooled to give a suspension (I) containing microcapsules in which the model fragrance material A was encapsulated with amorphous silica.

The encapsulation ratio of hexyl acetate, tetrahydrolinalol, hexylcynnamaldehyde and methyl dihydrojasmonate in microcapsules contained in the suspension (I) was 78%, 83%, 96%, and 92%, respectively.

### Example 2

### (Step 1)

150 g of an aqueous dispersion was prepared in the same manner as in (Step 1) in Example 1.

### (Step 2)

25 g of the aqueous dispersant prepared in the step 1 was diluted with 75 g of water added thereto (dilution ratio 4 times). Next, while the liquid temperature was kept at 30°C, 6.0 g of TEOS was dropwise added via the above-mentioned dropping pump taking 425 minutes. After the dropwise addition, this was kept stirred for further 18 hours and then cooled to give a suspension (II) containing microcapsules in which the model fragrance material A was encapsulated with amorphous silica.

The encapsulation ratio of hexyl acetate, tetrahydrolinalol, hexylcynnamaldehyde and methyl dihydrojasmonate in microcapsules contained in the suspension (II) was 79%, 80%, 90% and 92%, respectively.

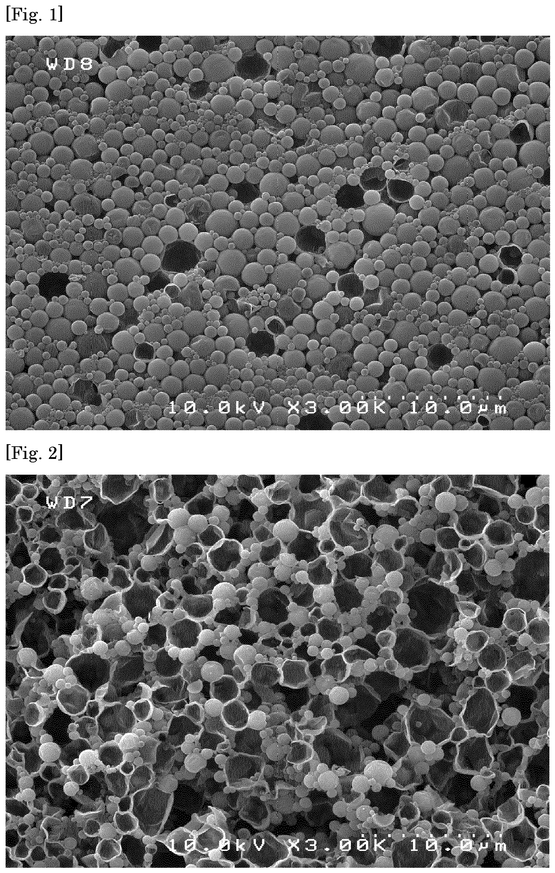

The resultant microcapsules were observed with SEM. The results are shown in Fig. 1.

### Example 3

### (Step 1)

150 g of an aqueous dispersion was prepared in the same manner as in (Step 1) in Example 1.

### (Step 2)

25 g of the aqueous dispersant prepared in the step 1 was diluted with 25 g of water added thereto (dilution ratio 2 times). Next, while the liquid temperature was kept at 30°C, 6.0 g of TEOS was dropwise added via the above-mentioned dropping pump taking 425 minutes. After the dropwise addition, this was kept stirred for further 18 hours and then cooled to give a suspension (III) containing microcapsules in which the model fragrance material A was encapsulated with amorphous silica.

The encapsulation ratio of hexyl acetate, tetrahydrolinalol, hexylcynnamaldehyde and methyl dihydrojasmonate in microcapsules contained in the suspension (III) was 63%, 68%, 85% and 80%, respectively.

### Example 4

### (Step 1)

150 g of an aqueous dispersion was prepared in the same manner as in (Step 1) in Example 1.

### (Step 2)

25 g of the aqueous dispersant prepared in the step 1 was diluted with 75 g of water added thereto (dilution ratio 4 times). Next, while the liquid temperature was kept at 30°C, 1.0 g of TEOS was dropwise added via the above-mentioned dropping pump taking 71 minutes. After the dropwise addition, this was kept stirred for further 18 hours and then cooled to give a suspension (IV) containing microcapsules in which the model fragrance material A was encapsulated with amorphous silica.

The encapsulation ratio of hexyl acetate, tetrahydrolinalol, hexylcynnamaldehyde and methyl dihydrojasmonate in microcapsules contained in the suspension (IV) was 74%, 77%, 90% and 79%, respectively.

### Example 5

### (Step 1)

150 g of an aqueous dispersion was prepared in the same manner as in (Step 1) in Example 1.

### (Step 2)

As a nonionic polymer, Polyvinyl Pyrrolidone K30 (tradename by FUJIFILM Wako Pure Chemical Corporation) was diluted with ion-exchanged water to prepare an aqueous polyvinyl pyrrolidone (PVP) solution containing 3% by mass of the active ingredient.

2 g of the aqueous 3 mass% PVP solution (active ingredient 0.06 g) and 73 g of water were added to 25 g of the aqueous dispersant prepared in the step 1 for dilution (dilution ratio 4 times). Next, while the liquid temperature was kept at 30°C, 6.0 g of TEOS was dropwise added via the above-mentioned dropping pump taking 425 minutes. After the dropwise addition, this was kept stirred for further 18 hours and then cooled to give a suspension (V) containing microcapsules in which the model fragrance material A was encapsulated with amorphous silica.

The encapsulation ratio of hexyl acetate, tetrahydrolinalol, hexylcynnamaldehyde and methyl dihydrojasmonate in microcapsules contained in the suspension (V) was 88%, 97%, 85% and 82%, respectively.

### Example 6

### (Step 1)

150 g of an aqueous dispersion was prepared in the same manner as in (Step 1) in Example 1.

### (Step 2)

As a nonionic polymer, hydroxypropyl cellulose (by FUJIFILM Wako Pure Chemical Corporation) was diluted with ion-exchanged water to prepare an aqueous hydroxypropyl cellulose (HPC) solution containing 3% by mass of the active ingredient.

2 g of the aqueous 3 mass% HPC solution (active ingredient 0.06 g) and 73 g of water were added to 25 g of the aqueous dispersant prepared in the step 1 for dilution (dilution ratio 4 times). Next, while the liquid temperature was kept at 30°C, 6.0 g of TEOS was dropwise added via the above-mentioned dropping pump taking 425 minutes. After the dropwise addition, this was kept stirred for further 18 hours and then cooled to give a suspension (VI) containing microcapsules in which the model fragrance material A was encapsulated with amorphous silica.

The encapsulation ratio of hexyl acetate, tetrahydrolinalol, hexylcynnamaldehyde and methyl dihydrojasmonate in microcapsules contained in the suspension (V) was 90%, 91%, 89% and 85%, respectively.

### Example 7

### (Step 1)

150 g of an aqueous dispersion was prepared in the same manner as in (Step 1) in Example 1.

### (Step 2)

2 g of the aqueous 3 mass% Merquat 100 solution (active ingredient 0.06 g) and 273 g of water were added to 25 g of the aqueous dispersion prepared in the step 1 for dilution (dilution ratio 12 times). Next, while the liquid temperature was kept at 30°C, 6.0 g of TEOS was dropwise added via the above-mentioned dropping pump taking 425 minutes. After the dropwise addition, this was kept stirred for further 18 hours, and then cooled to give a suspension (VII) containing microcapsules in which the model fragrance material A was encapsulated with amorphous silica.

The encapsulation ratio of hexyl acetate, tetrahydrolinalol, hexylcynnamaldehyde and methyl dihydrojasmonate in microcapsules contained in the suspension (VII) was 54%, 60%, 78%, and 75%, respectively.

### Example 8

### (Step 1)

1.67 g of ΓaUARTAMIN 60W (tradename by Kao Corporation, cetyltrimethylammonium chloride, active ingredient: 30% by mass) was diluted with 98.33 g of ion-exchanged water to give an aqueous solution. An organic phase prepared by mixing 40 g of the model fragrance material B and 10 g of tetraethoxysilane (TEOS) was added to the aqueous solution, and the mixture liquid was emulsified using a homomixer set at a rotation number of 5,000 rpm. At that time, the volume-average particle diameter of the emulsified drops was 2.25 pm. This was controlled to have a pH of 3.8 using an aqueous 0.1 mol/L sodium hydroxide solution, then transferred to a separable flask equipped with a stirring blade and a cooling device, and while the liquid temperature was kept at 30°C, this was stirred at 160 rpm for 17 hours to give 150 g of an aqueous dispersion containing capsules (1-8) each having a core and a shell.

### (Step 2)

2 g of the aqueous 3 mass% Merquat 100 solution (active ingredient 0.06 g) and 73 g of water were added to 25 g of the aqueous dispersion prepared in the step 1 for dilution (dilution ratio 4 times). Next, while the liquid temperature was kept at 30°C, 6.0 g of TEOS was dropwise added via the above-mentioned dropping pump taking 425 minutes. After the dropwise addition, this was kept stirred for further 18 hours, and then cooled to give a suspension (VIII) containing microcapsules in which the model fragrance material B was encapsulated with amorphous silica.

The encapsulation ratio of citronellol, geraniol, hexyl butyrate and linalyl acetate in microcapsules contained in the suspension (VIII) was 77%, 79%, 83%, and 85%, respectively.

### Comparative Example 1

### (Step 1)

1.74 g of ΓaUARTAMIN 60W (tradename by Kao Corporation, cetyltrimethylammonium chloride, active ingredient: 30% by mass) was diluted with 98.26 g of ion-exchanged water to give an aqueous solution. An organic phase prepared by mixing 40 g of the model fragrance material A and 10 g of tetraethoxysilane (TEOS) was added to the aqueous solution, and the mixture liquid was emulsified using a homomixer set at a rotation number of 8,500 rpm. At that time, the volume-average particle diameter of the emulsified drops was 2.7 pm. This was controlled to have a pH of 3.9 using an aqueous 1N sodium hydroxide solution, then transferred to a separable flask equipped with a stirring blade and a cooling device, and while the liquid temperature was kept at 30°C, this was stirred at 160 rpm for 17 hours to give 150 g of an aqueous dispersion containing capsules (1-C1) each having a core and a shell.

### (Step 2)

While the liquid temperature of 150 g of the aqueous dispersion prepared in the step 1 was kept at 30°C, 5.7 g of TEOS was dropwise added thereto via the above-mentioned dropping pump taking 320 minutes. After the dropwise addition, this was kept stirred for further 18 hours, and then cooled to give a suspension (CI) containing microcapsules in which the model fragrance material A was encapsulated with amorphous silica.

The encapsulation ratio of hexyl acetate, tetrahydrolinalol, hexylcynnamaldehyde and methyl dihydrojasmonate in microcapsules contained in the suspension (CI) was 75%, 79%, 96%, and 85%, respectively.

The resultant microcapsules were observed with SEM. The results are shown in Fig. 2.

### Comparative Example 2

### (Step 1)

0.83 g of ΓaUARTAMIN 60W (tradename by Kao Corporation, cetyltrimethylammonium chloride, active ingredient: 30% by mass) was diluted with 274.17 g of ion-exchanged water to give an aqueous solution. An organic phase prepared by mixing 20 g of the model fragrance material A and 5 g of tetraethoxysilane was added to the aqueous solution, and the mixture liquid was emulsified using a homomixer set at a rotation number of 8,200 rpm. At that time, the volume-average particle diameter of the emulsified drops was 1.2 pm. This was controlled to have a pH of 3.8 using an aqueous 1N sodium hydroxide solution, then transferred to a separable flask equipped with a stirring blade and a cooling device, and while the liquid temperature was kept at 30°C, this was stirred at 160 rpm for 24 hours to give 300 g of an aqueous dispersion containing capsules (1-C2) each having a core and a shell.

### (Step 2)

While the liquid temperature of 150 g of the aqueous dispersion prepared in the step 1 was kept at 30°C, 11.9 g of TEOS was dropwise added thereto via the above-mentioned dropping pump taking 420 minutes. After the dropwise addition, this was kept stirred for further 19 hours, and then cooled to give a suspension (CII) containing microcapsules in which the model fragrance material A was encapsulated with amorphous silica.

The encapsulation ratio of hexyl acetate, tetrahydrolinalol, hexylcynnamaldehyde and methyl dihydrojasmonate in microcapsules contained in the suspension (CII) was 37%, 42%, 53%, and 41%, respectively.

The encapsulation ratio of each fragrance material component in the microcapsules formed in Comparative Example 2 was an extremely low value, and therefore the test mentioned below for evaluation of long-term retentiveness of the fragrance material component encapsulated in the microcapsules in a softener was omitted.

In Examples and Comparative Examples, the total amount [g] of the organic compound and TEOS in the step 1; the total amount of the aqueous dispersion before addition of TEOS (namely, the total amount of the dispersion in diluting water) [g]; the total amount [g] of the organic compound and TEOS in the step 1 relative to the total amount [g] of the aqueous dispersion before addition of TEOS in the step 2 (% by mass); the amount added of TEOS in the step 2 relative to the amount of the organic compound in the step 1 (% by mass); and the total amount added of TEOS relative to the amount of the organic compound in the step 1 (% by mass) are shown in the following Table 3.

**Table 3-1**

| | Step 1 | | | | | Step 2 | | | | | |
|---|---|---|---|---|---|---|---|---|---|---|---|
| | Formulation | | | | | Formulation | | | | | Dilution ratio (times) |
| | Amount added of ion-exchange d water [g] | Surfactant (*1) | Organic compound | | Amount added of TEOS [g] | Amount used of aqueous dispersion prepared in step 1 [g] | Amount added of ion-exchang ed water [g] | Organic polymer compound | | Amount added of TEOS [g] | |
| | | Amount added [g] | Kind | Amount added [g] | | | | Kind | Amount added of aqueous solution [g] | | |
| Example 1 | 98.33 | 1.67 | Fragrance material A | 40 | 10 | 25 | 73 | Merquat 100 | 2 | 6.0 | 4 |
| Example 2 | 98.33 | 1.67 | Fragrance material A | 40 | 10 | 25 | 75 | no | - | 6.0 | 4 |
| Example 3 | 98.33 | 1.67 | Fragrance material A | 40 | 10 | 25 | 25 | no | - | 6.0 | 2 |
| Example 4 | 98.33 | 1.67 | Fragrance material A | 40 | 10 | 25 | 75 | no | - | 1.0 | 4 |
| Example 5 | 98.33 | 1.67 | Fragrance material A | 40 | 10 | 25 | 73 | PVP | 2 | 6.0 | 4 |
| Example 6 | 98.33 | 1.67 | Fragrance material A | 40 | 10 | 25 | 73 | HPC | 2 | 6.0 | 4 |
| Example 7 | 98.33 | 1.67 | Fragrance material A | 40 | 10 | 25 | 273 | Merquat 100 | 2 | 6.0 | 12 |
| Example 8 | 98.33 | 1.67 | Fragrance material B | 40 | 10 | 25 | 73 | Merquat 100 | 2 | 6.0 | 4 |
| Comparative Example 1 | 98.26 | 1.74 | Fragrance material A | 40 | 10 | 150 | - | no | - | 5.7 | - |
| Comparative Example 2 | 274.17 | 0.83 | Fragrance material A | 20 | 5 | 150 | - | no | - | 11.9 | - |

**Table 3-2**

| | Total amount of organic compound and TEOS in step 1 [g] (*2) | Total amount of aqueous dispersion before addition of TEOS in step 2 [g] | Total amount of organic compound and TEOS in step 1 relative to total amount of aqueous dispersion before addition of TEOS in step 2 [mass%] (*3) | Amount added of TEOS in step 2 relative to amount of organic compound in step 1 [mass%) | Total amount added of TEOS relative to amount of organic compound in step 1 [mass%] |
|---|---|---|---|---|---|
| Example 1 | 8.33 | 100 | 8.3 | 90 | 115 |
| Example 2 | 8.33 | 100 | 8.3 | 90 | 115 |
| Example 3 | 8.33 | 50 | 16.7 | 90 | 115 |
| Example 4 | 8.33 | 100 | 8.3 | 15 | 40 |
| Example 5 | 8.33 | 100 | 8.3 | 90 | 115 |
| Example 6 | 8.33 | 100 | 8.3 | 90 | 115 |
| Example 7 | 8.33 | 300 | 2.8 | 90 | 115 |
| Example 8 | 8.33 | 100 | 8.3 | 90 | 115 |
| Comparative Example 1 | 50 | 150 | 33.3 | 14 | 39 |
| Comparative Example 2 | 12.51 | 150 | 8.3 | 119 | 144 |

| | | | | | |
|---|---|---|---|---|---|
| Notes in Table 3 are as follows. *1: QUARTAMIN 60W (active ingredient: 30% by mass) *2: Amount [g] calculated by proportional conversion of the amount of the aqueous dispersion containing the capsules (1) formed in the step 1 and subjected to the step 2, from the total amount [g] of the organic compound and TEOS used for preparing the aqueous dispersion containing the capsules (1) in the step 1. *3: Total amount [g] (mass%) of the organic compound and TEOS in the step 1, relative to the total amount [g] of the aqueous dispersion before addition of TEOS in the step 2. | | | | | |

### <Evaluation> (Evaluation of long-term retentiveness of fragrance material component encapsulated in microcapsules in softener)

### [Preparation of softener for evaluation)

A suspension containing the microcapsules formed in Examples 1 to 8 and Comparative Example 1 was added to a softener base material having the composition shown in the following Table 4 to prepare a softener for evaluation.

At that time, the content of the encapsulated fragrance material was controlled to be 0.5% by mass.

**Table 4**

| Softener Base Material(*1) | Amount blended (part by mass) |
|---|---|
| Cationic softener base material(*2) | 12 |
| Polyoxyethylene(40) lauryl ether | 3.5 |
| Calcium chloride | 0.2 |
| Ethylene glycol | 1.6 |
| Proxel BDN(*3) | 0.01 |
| Methyl glycine diacetate trisodium | 0.01 |
| Ion-exchanged water | 82.68 |
| Total | 100 |

| | |
|---|---|
| Notes in Table 4 are as follows. *1: Added to make pH of softener base material 3.2. *2: Ester amine prepared by reacting plant fatty acid and triethanol amine in a ratio of 1.65/1 mol was quaternized with dimethyl sulfate in a known method. *3: by LONZA Japan, Ltd. | |

### (Evaluation method for long-term retentiveness of fragrance material component)

The softener containing the microcapsule suspension was put into a screw tube, sealed up therein and stored at 40°C.

After the preparation, that is, 21 days after the start of storage, the screw tube was taken out, and 100 mg of the softener was scooped out with a syringe, diluted with 10 g of ion-exchanged water, and then made to pass through a membrane filter (by Millipore Corporation, tradename "Omnipore", Model Code "JAWP04700") to collect the capsules on the membrane filter.

Further, on the membrane filter, the microcapsules were washed with 10 mL of ion-exchanged water and then with 10 mL of hexane, and the microcapsules were immersed in 2 mL of acetonitrile containing tridecane in a concentration of 10 pg/ml, as an internal standard, and irradiated with ultrasonic waves for 60 minutes using an ultrasonic irradiator (by Branson Corporation, Model "5510") under the condition of an output of 180 W and an oscillation frequency of 42 kHz to thereby elute the fragrance material out of the microcapsules. The resultant solution was again made to pass through a membrane filter (by Toyo Roshi Kaisha, Ltd., tradename "DISMIC", Model Code "13JP020AN"), and thereafter the fragrance material contained in the solution was identified through gas chromatography to determine the amount of the fragrance material component included in the suspension. The amount is referred to as α. The fragrance material retentiveness was calculated according to the following formula. The evaluation results in Examples 1 to 8 and Comparative Example 1 are shown in the following Table 5.

Fragrance material retentiveness (%) = {(amount α of fragrance material component encapsulated in microcapsules after storage)/(amount β of fragrance material component contained in 100 mg of softener)} × 100

The amount β of the fragrance material component in the above formula was calculated from the formulation of the model fragrance material, the encapsulation ratio of the fragrance material, and the blending amount of the microcapsules used in preparing the softener.

**Table 5**

| | Evaluation Results (fragrance material retentiveness (%) after storage for 21 days at 40°C) | | | | | | | |
|---|---|---|---|---|---|---|---|---|
| | Model Fragrance Material A | | | | Model Fragrance Material B | | | |
| | Hexyl acetate | Tetrahydrolinalol | hexylcynnam aldehyde | methyl dihydro-iasmonate | citronellol | geraniol | hexyl butyrate | linalyl acetate |
| Example 1 | 49 | 60 | 45 | 62 | - | - | - | - |
| Example 2 | 53 | 68 | 56 | 75 | - | - | - | - |
| Example 3 | 17 | 25 | 16 | 28 | - | - | - | - |
| Example 4 | 53 | 60 | 30 | 55 | - | - | - | - |
| Example 5 | 54 | 72 | 52 | 59 | - | - | - | - |
| Example 6 | 55 | 66 | 61 | 72 | - | - | - | - |
| Example 7 | 34 | 41 | 19 | 44 | - | - | - | - |
| Example 8 | - | - | - | - | 61 | 57 | 60 | 68 |
| Comparative Example 1 | 5 | 19 | 18 | 15 | - | - | - | - |

As obvious from Table 5, it is known that the microcapsules prepared by the method of the present invention can hold an organic compound as an effective ingredient of a fragrance material etc. for a long period of time. Accordingly, the microcapsules prepared by the method of the present invention are favorably used for various products blended with an effective ingredient of a fragrance material, etc.

Comparison is made between Fig. 1 and Fig. 2 that are SEM pictures of the microcapsules obtained in Example 2 and Comparative Example 1, respectively. It is known that the microcapsules obtained in Example 2 cracked little and held good spherical forms.

From the results of the encapsulation ratio of each fragrance material component immediately after production, it is considered that in Comparative Example 1, spherical microcapsules could be produced like in Example 2, however, it is considered that in Comparative Example 1, the shell strength was lower than in Example 2 and therefore caused cracking under the treatment condition for SEM observation in the SEM picture of the microcapsules as shown in Fig. 2.

## Claims

1. A method for producing microcapsules each comprising a shell that comprises silica as a constituent ingredient, and a core that comprises an organic compound inside the shell, which comprises:
a step 1 of emulsifying an organic phase comprising an organic compound and a tetraalkoxysilane in an aqueous phase comprising a surfactant, followed by sol-gel reaction under an acidic condition to form capsules (1) each comprising a core and a shell, thereby giving an aqueous dispersion that comprises the capsules (1), and
a step 2 of diluting the aqueous dispersion prepared in the step 1 with water added thereto, and further adding a tetraalkoxysilane and subjecting the obtained mixture to sol-gel reaction to form a shell that encapsulates the capsule (1), thereby giving microcapsules, and wherein:
the dilution in the step 2 is carried out in such a manner that the total amount of the organic compound and the tetraalkoxysilane in the step 1 is 18% by mass or less relative to 100% by mass of the total amount of the aqueous dispersion before addition of the tetraalkoxysilane in the step 2.

2. The method for producing microcapsules according to claim 1, wherein in the step 2, the dilution ratio is 2 times or more and 20 times or less.

3. The method for producing microcapsules according to claim 1 or 2, wherein in the step 2, an organic polymer compound is further added to the aqueous dispersion formed in the step 1.

4. The method for producing microcapsules according to claim 3, wherein the organic polymer compound is at least one selected from the group consisting of a polydiallyldimethylammonium salt and a copolymer thereof, a polyvinyl pyrrolidone and a hydroxypropyl cellulose.

5. The method for producing microcapsules according to any one of claims 1 to 4, wherein the amount of tetraalkoxysilane to be added in the step 2 is 10% by mass or more and 200% by mass or less relative to 100% by mass of the amount of the organic compound in the step 1.

6. The method for producing microcapsules according to any one of claims 1 to 5, wherein the tetraalkoxysilane to be used in the step 1 is at least one selected from the group consisting of tetramethoxysilane, tetraethoxysilane and tetraisopropoxysilane.

7. The method for producing microcapsules according to any one of claims 1 to 6, wherein the tetraalkoxysilane to be used in the step 2 is at least one selected from the group consisting of tetramethoxysilane, tetraethoxysilane and tetraisopropoxysilane.

8. The method for producing microcapsules according to any one of claims 1 to 7, wherein the organic compound is at least one selected from the group consisting of a fragrance material, a fragrance precursor, an oil, an antioxidant and a solvent.

## Patentansprüche

1. Verfahren zur Herstellung von Mikrokapseln, die jeweils eine Hülle, umfassend Siliciumoxid als Bestandteil, und einen Kern, umfassend eine organische Verbindung, innerhalb der Hülle umfassen, wobei das Verfahren umfasst:
einen Schritt 1 des Emulgierens einer organischen Phase, umfassend eine organische Verbindung und ein Tetraalkoxysilan, in einer wässrigen Phase, die ein Tensid umfasst, gefolgt von einer Sol-Gel-Reaktion unter sauren Bedingungen, um Kapseln (1) zu bilden, die jeweils einen Kern und eine Hülle umfassen, wodurch eine wässrige Dispersion erhalten wird, die die Kapseln (1) umfasst, und
einen Schritt 2 des Verdünnens der in Schritt 1 hergestellten wässrigen Dispersion mit hinzugefügtem Wasser und des weiteren Hinzufügens eines Tetraalkoxysilans und des Unterziehens der erhaltenen Mischung einer Sol-Gel-Reaktion, um eine Hülle zu bilden, die die Kapsel (1) einkapselt, wodurch Mikrokapseln erhalten werden, und wobei:
das Verdünnen in Schritt 2 so durchgeführt wird, dass die Gesamtmenge der organischen Verbindung und des Tetraalkoxysilans in Schritt 1 18 Massen-% oder weniger, bezogen auf 100 Massen-% der Gesamtmenge der wässrigen Dispersion vor dem Hinzufügen des Tetraalkoxysilans in Schritt 2, beträgt.

2. Verfahren zur Herstellung von Mikrokapseln gemäß Anspruch 1, wobei in Schritt 2 das Verdünnungsverhältnis 2-mal oder mehr und 20-mal oder weniger beträgt.

3. Verfahren zur Herstellung von Mikrokapseln gemäß Anspruch 1 oder 2, wobei in Schritt 2 ferner eine organische Polymerverbindung zu der in Schritt 1 gebildeten wässrigen Dispersion gegeben wird.

4. Verfahren zur Herstellung von Mikrokapseln gemäß Anspruch 3, wobei die organische Polymerverbindung mindestens eine ist, die aus der Gruppe ausgewählt ist, die aus einem Polydiallyldimethylammoniumsalz und einem Copolymer davon, einem Polyvinylpyrrolidon und einer Hydroxypropylcellulose besteht.

5. Verfahren zur Herstellung von Mikrokapseln gemäß einem der Ansprüche 1 bis 4, wobei die Menge an Tetraalkoxysilan, die in Schritt 2 zugegeben wird, 10 Massen-% oder mehr und 200 Massen-% oder weniger, bezogen auf 100 Massen-% der Menge der organischen Verbindung in Schritt 1, beträgt.

6. Verfahren zur Herstellung von Mikrokapseln gemäß einem der Ansprüche 1 bis 5, wobei das in Schritt 1 zu verwendende Tetraalkoxysilan mindestens eines ist, das aus der Gruppe ausgewählt ist, die aus Tetramethoxysilan, Tetraethoxysilan und Tetraisopropoxysilan besteht.

7. Verfahren zur Herstellung von Mikrokapseln gemäß einem der Ansprüche 1 bis 6, wobei das in Schritt 2 zu verwendende Tetraalkoxysilan mindestens eines ist, das aus der Gruppe ausgewählt ist, die aus Tetramethoxysilan, Tetraethoxysilan und Tetraisopropoxysilan besteht.

8. Verfahren zur Herstellung von Mikrokapseln gemäß einem der Ansprüche 1 bis 7, wobei die organische Verbindung mindestens eine ist, die aus der Gruppe ausgewählt ist, die aus einem Duftstoff, einem Duftstoffvorläufer, einem Öl, einem Antioxidationsmittel und einem Lösungsmittel besteht.

## Revendications

1. Procédé de production de microcapsules comprenant chacune une enveloppe qui comprend de la silice en tant qu'ingrédient constitutif, et un noyau qui comprend un composé organique à l'intérieur de l'enveloppe, qui comprend :
une étape 1 d'émulsification d'une phase organique comprenant un composé organique et un tétraalkoxysilane en phase aqueuse comprenant un tensioactif, suivie par une réaction sol-gel en condition acide pour former des capsules (1) chacune comprenant un noyau et une enveloppe, donnant ainsi une dispersion aqueuse qui comprend les capsules (1), et
une étape 2 de dilution de la dispersion aqueuse préparée dans l'étape 1 avec ajout d'eau, et ajout en outre d'un tétraalkoxysilane et soumission du mélange obtenu à la réaction sol-gel pour former une enveloppe qui encapsule la capsule (1), donnant ainsi des microcapsules, et dans lequel :
la dilution dans l'étape 2 est menée de telle manière que la quantité totale du composé organique et du tétraalkoxysilane dans l'étape 1 est 18 % en masse ou moins par rapport à 100 % en masse de la quantité totale de la dispersion aqueuse avant l'ajout du tétraalkoxysilane dans l'étape 2.

2. Procédé de production de microcapsules selon la revendication 1, dans lequel dans l'étape 2, le rapport de dilution est 2 fois ou plus et 20 fois ou moins.

3. Procédé de production de microcapsules selon la revendication 1 ou la revendication 2, dans lequel dans l'étape 2, un composé polymère organique est en outre ajouté à la dispersion aqueuse formée dans l'étape 1.

4. Procédé de production de microcapsules selon la revendication 3, dans lequel le composé polymère organique est au moins l'un choisi parmi le groupe consistant en un sel de polydiallyldiméthylammonium et un copolymère de celui-ci, un pyrrolidone de polyvinyle et une hydroxypropyl cellulose.

5. Procédé de production de microcapsules selon l'une quelconque des revendications 1 à 4, dans lequel la quantité de tétraalkoxysilane à ajouter dans l'étape 2 est 10 % en masse ou plus et 200 % en masse ou moins par rapport à 100 % en masse de la quantité du composé organique dans l'étape 1.

6. Procédé de production de microcapsules selon l'une quelconque des revendications 1 à 5, dans lequel le tétraalkoxysilane à utiliser dans l'étape 1 est au moins un choisi parmi le groupe consistant en tétraméthoxysilane, tétraéthoxysilane et tétraisopropoxysilane.

7. Procédé de production de microcapsules selon l'une quelconque des revendications 1 à 6, dans lequel le tétraalkoxysilane à utiliser dans l'étape 2 est au moins un choisi parmi le groupe consistant en tétraméthoxysilane, tétraéthoxysilane et tétraisopropoxysilane.

8. Procédé de production de microcapsules selon l'une quelconque des revendications 1 à 7, dans lequel le composé organique est au moins un choisi parmi le groupe consistant en un matériau de fragrance, un précurseur de fragrance, une huile, un antioxydant et un solvant.
